# EUROPEAN PATENT APPLICATION

(11) **EP 3 112 105 A1**
(43) Date of publication of application: **04.01.2017**
(21) Application number: 15754710.0
(22) Date of filing: 27.02.2015
(51) Int. Cl.: B27K 7/00

(54) **METHOD, DEVICE AND FACILITY FOR INDUCING CONTROLLED BREATHING IN CORKS IN ORDER TO ELIMINATE HARMFUL SUBSTANCES OR GASES**

(30) Priority: 27.02.2014 ES 201430267
(71) Applicant: Godoy Varo, José Luis, 17244 Cassà de la Selva (Girona) (ES)
(72) Inventor: GODOY VARO, Jose Luis, 17244 Cassà de la Selva (Girona) (ES); PARRAMÓN AMETLLER, Francesc, 17244 Cassà de la Selva (Girona) (ES)
(86) International application number: PCT/ES2015/070139
(87) International publication number: WO 2015/128531

(57) **Abstract**

The method consists of inducing a "controlled breathing" method to the cork, in its natural state or in all and each of its stages of development. Placing the item to be treated in a sealed container or circuit. Monitoring and controlling the pressure differential between the inside and outside, with reference to the consumption of ozone and / or oxygen initially provided and / or to the generated chemical compounds or, allowing sensory improvement and elimination of trichloroanisole (TCA) 2- 4-6 by using this treatment.

## Description

### Field of the technique

The treatment method is applicable to all types of compounds, organic substances and cork products, in their natural state or in each and every one of its phases of development or manufacture.

The technology sector includes the induction of "Controlled Breathing" to cork in an airtight container at a temperature of less than 20 °C for a controlled time, and from predetermined analytical parameters. By "breathing", we understand the succession of inhalations and exhalations caused by mechanical elements with input and extraction of at least one part of oxidizing gas, which is induced and controlled by a computer that processes data on chemical and thermal parameters captured inside the container. After each gas injection (inhalation), its gradual decline due to the interaction between oxidant gas and cork is measured, up to pre-set limits, when the controlled vacuum and/or sweep system starts (exhalation) to extract the newly generated gases, clean up the environment, and recover the predetermined pressure with at least one oxidizing gas. Now, a cycle is completed, one that will continue with a new inhalation and so on, until treatment is completed. Periodically, as required by the process, but always at least once, it is recommended to apply a temperature with higher values than the initial one. The cyclical repetition of these phases, which can last hours or days, is what we call "Controlled Breathing".

"Controlled Breathing" includes the following steps:
a) The gas is introduced into the sealed container by a pressure differential.
b) In parallel to step a), the amount present in the container of at least one of the chemicals supplied and/or generated in the reaction, is monitored and/or controlled so that, once an increase or decrease from a predetermined value is detected, it goes to the next phase.
c) Next, we continue by extracting at least one of the substances provided and/or generated in the reaction, for a predetermined time and/or depending on the comparison of the concentration values detected regarding the default ones.
d) Steps a), b) and c) are repeated cyclically when treatment is required.
e) Steps a), b) and c) are performed keeping the container at temperatures ranging between 1 and 20 ºC.
f) However, the process always requires that at least one steam and/or temperature cycle takes place at temperatures higher than 20 °C to administered gas or gases and dissolved in the cork to react, causing eliminate unwanted compounds. For heat to impinge more easily and homogeneity in the porosities and cavities of the cork, a prior vacuum cycle can be administered.

The end result is a series of significant changes in the cork, depending on the nature of the contaminant, but with the common denominator of removing substances and compounds that the cork could transmit; for example, 2-4-6 trichloroanisole (TCA), a noxious gas that contaminated cork stoppers transmit to beverages they clog, causing the product to deteriorate, also causing significant economic losses, particularly in the wine sector.

### Status of the prior technique

A method is known to dry and cure solid or semisolid products, Patent PCT / ES2012 / 070612 WO2012 / 021086, belonging to the same inventor of the present.

Both patents overlap in the method; that is, the introduction of an oxidizing gas treatment to the means of treatment by a pressure differential, and the subsequent extraction of the gases resulting from the reaction by vacuum. It is important to highlight that the control on inputs and outputs is performed by predetermined times. Due to the heterogeneity of the compounds to treat, it is difficult to accurately control the process, so you cannot successfully know the times of application times -for excess or by default-, which prevents the insurance of optimal process results.

The main innovations reside in the facility within the treatment container and/or in areas of extraction of gas sensors that allow:
a). - Control and monitoring of the decrease of the initial oxidizing agent placed to decide when to move to the next step.
b). - Controlling and monitoring of the increase or decrease of at least one chemical substance generated in the reaction, to decide when to move to the next step.
c). - Control and input of temperature in two phases, one allowing the dissolution of the oxidizing agent introduced, and the other its reaction.

In all three cases more detailed and concise information about the changes that occur during the process is obtained.

It has been found that if the cork is contaminated, the higher the contamination, the higher the consumption of the applied oxidizing agent.

Therefore, it is in our interest to:
1. - Better control the time in treatment cycles, lengthening or shortening each of the phases based on the consumption of oxidizing gas (oxygen, ozone, etc.) introduced by a pressure differential.
2. - Adjust these times to each type of cork or product manufactured with it according to their shape, volume and level of contamination, seeking the optimal scale,
3. - Control gas or chemicals caused in reaction, lengthening or shortening extraction times.
4. - And simultaneously control the sweep that is induced which allows lengthening or shortening of the extraction time of the reaction components and/or the oxidizing agent inlet.
5. - Control the dissolution and reaction temperature systematically.

Therefore, the process proceeds to control itself according to the consumed amount of oxidizing agents or chemicals generated, rather than by predetermined times.

Referring to prior technique processes, currently no proposal is known, where to pass pressure or sweeping differential cycles, the control comes from the oxidant gas consumption contributed to the atmosphere of the treatment. Nor is a technique in which the reaction compounds generated are controlled, determining the increase or decrease by sensors or analysers of chemicals, like gases chromatographs, to use this information to control of the process. Likewise, processes which use the above combinations with dissolution and reaction controlled temperatures, are unknown, the same as the pressure oscillations, with the objective of altogether facilitate the removal of contaminating gases adhered to the cork.

### Disclosure of the invention

A tank, vessel, chamber or sealed container with at least one gate that communicates with the outer atmosphere, prepared for retention of applied pressures, equipped inside with at least one gas sensor, -02, 03 for example, and/or other chemicals, is necessary. Its shape, size, stiffness or elasticity will adapt to the elements that are entered in it to be treated, this being raw cork, cork stoppers, discs, granules, etc.

The container with cork to be treated is loaded, and a process at a temperature with values between 1 and 20 is applied. An oxidizing agent, gas or combination of gases, containing at least one part of oxidizing agent (oxygen or ozone), is also introduced, by differential pressure. Subsequently the internal pressure is increased; as required by the process, this increase may be accompanied by pressure oscillations within the range of atmospheric values, or above and below these. This is the inhalation phase. It constantly monitors and measures the internal consumption of oxidizing agent introduced; although it could also be interesting to control other gases generated by reactions between the administered gas and the cork. The information obtained is monitored and processed with a specific computer program that compares it with the initial values. When the default values for each treatment levels are reached, it proceeds to apply a vacuum and/or sweeping, or a combination of both, to extract the newly generated gases and clean the environment inside the container. This is the Exhalation phase.

The cyclical repetition of the phases of "Inhalation" and "Exhalation" creates the "Breathing" effect, which is later "controlled" thanks to closely monitoring the evolution of the relative amounts of gases (02, 03 and others) or compounds within the vessel where the treatment is performed. The processing of data collected directs and sets the pace of the management process and extraction. For example, 02 consumption indicates that the material is absorbing it, it reacts and transforms with it; but an 02 minimum relative presence value has been programmed from which it is necessary to remove from the recipient the reaction generated gases or compounds (Exhalation), and restart the cycle (Inhalation).

The information obtained is also very valuable in other aspects: it allows you to set and confirm the end of treatment, because normally a low consumption of 02 or 03 will establish precisely when to cease the process of "Controlled Breathing"; it allows verification that the evolution of the process goes as planned; it allows a more precise control over the method if the group of substances to be treated are not homogeneous in volume or weight; in general, it can shorten labour, and save on energy consumption. Sensor chemical readings also provide information based on which, by software, if necessary to add additional time between cycles, is decided.

You can also apply a range of pressure oscillations, which will allow the applied gases to be introduced, dissolve and react more easily in the cork. These pressure oscillations are made at about the same range values, above or below atmospheric pressure.

Although, initially, the temperature inside the container shall not exceed 20 °C, it may be desirable to apply higher temperatures to facilitate the reaction of dissolved chemicals.

Another possibility would be to introduce devices that generate mechanical vibrations, applied to the product or in the area of the air chamber. They would be controlled by pneumatic devices, frequency inverters and/or devices to control their electrical current, which would regulate their frequency (Hz). The vibrations can be generated continuously or spaced, in each of the "Inhalation", "Exhalation", and "Sweep" cycles, additional time between cycles and/or a combination of these. These mechanical vibrations can improve treatment and shorten implementation times.

Here we see a more detailed description of the steps of the invention's method:
The "Inhalation" is achieved by the application into the container of a sweep or a positive pressure, which is equal, below or above atmospheric, using air from the outside surrounding atmosphere, from oxygen or ozone generators. Or a gas which is rich in oxygen, usually sold in containers or pressurized cylinders, or any combination with at least one of the above. Keep in mind that the higher the pressure applied, the higher the density of gases. For example, if we choose atmospheric gas, with approximately 21% of oxygen, at a pressure of 1,015 millibars of absolute pressure, it will have the same 21% of oxygen than if we had applied 2,100 millibars of absolute pressure, but the density of oxygen is higher. That is, comparing two trials of the same treatment, which used a level of higher pressure, will present a slower oxygen consumption (%). We will have to enter these parameters in the computer system that manages the Inhalation/ Exhalation process cycles.

There are several methods that introduce gas into a container. For this invention we want to manipulate and record the differential pressure between the interior and the exterior. We can achieve this in at least five ways:
a) Introducing gas into the container with the simple application of a higher relative pressure in the inside regarding the exterior. This option has the advantage of allowing a greater supply of oxygen or ozone as pressure is added because of the increased density of gases. Standing at pressure range values lower or higher than atmospheric, which will be beneficial to treat most substances.
b) Applying a controlled vacuum closed inside the container with the gate communicating with the outer, the pressure will lower to the desired levels; when the gate is opened and the vacuum is stopped, due to the differential pressure, the atmospheric air (with its share of oxygen or ozone generated) will enter, until the internal pressure stabilizes with the outside pressure, or to pre-programmed levels, closing the entrance gate in a controlled way-. This is the advantage of having the ability to communicate the interior volume of the container with the atmosphere outside by opening at least one of the container's gates to cause the recovery of the interior atmospheric pressure to default values.
c) An oxidizing agent can also be supplied when performing a controlled sweep gas from the atmosphere inside the container allowing its renewal for a pre-set time and/or to achieve predetermined values. At the same time, it controllably opens an outer door -preferably located in a substantially opposite are to the suction-vacuum, for the oxidizing gas to enter, flow and enrich the indoor environment, while reaction compounds are extracted, caused in the previous oxidation step. After a set time or via controlling the oxidizing agent introduced, the gate that generates the vacuum is closed and allowed to stabilize the inner atmosphere at a predetermined pressure, which can be the surrounding atmospheric one. This will leave the compound's to be treated contour rich in the oxidizing agent and provided with the combination of the gate closing which communicates with the outside and the administration of an overpressure to the middle, this one enters in the other.
d) Similarly, it is also feasible to introduce the oxidizing agent with administration of a higher pressure that the one inside or outside the container, while a gate opened to communicate with the outside -located preferably in the substantially opposite end of the admission-, thereby creating a sweeping overpressure inside the enclosure which will drag volatiles and leave the contour of the material to be treated rich in the oxidizing gas provided. The combination of the closure of the gate that communicates with the outside and the applying of a positive pressure will allow introducing the oxidizing agent to a particular pressure value or values to reach predetermined concentration. Note that at this stage or example, vacuum application is not necessary because of a controlled sweep of interior atmospheric gas, allowing renewal.
e) As it is also possible to recirculate the gas in the container forcing it to go through an ozone generator using a circulating pump; with this we achieve a higher volume of this gas in the container without inducing the above steps. Another advantage of this method is that as the gas is introduced into the cork, the low pressure, so it is necessary to introduce more gas to compensate for the loss. If this gas is atmospheric air, with its concentration of 02, a part will become 03 when passing through the ozone generator when recirculating in the container.

In the next phase only when the software has processed the data obtained by chemical sensors installed inside the container and, comparing them with the information programmed on minimum values 02, 03 and/or other gases or chemicals generated for each type of treated substance, it concludes that the "Inhalation" phase is over.

The "Exhalation" phase is achieved by applying a vacuum and/or sweeping the inside of the container at equal or below the range atmospheric pressures, preferably less than the internal pressure existing value. In this way, the generated gases are extracted from the container by the reaction between the cork and the oxidizing compound introduced into the phase of "Inhalation", especially a significant amount of C02. It is advisable to have the output of the extraction system in a ventilated and free from staff area.

Thanks to the chemicals sensors, controlled and cyclical repetition to the phases of "Inhalation" and "Exhalation" allows the treated cork to evolve dramatically and to desired levels. Multiple repetitions of these cycles until reaching final predetermined chemical parameters cause the effect we call "Controlled Breathing".

It is also possible to apply a sweep to the internal atmosphere gas between "Inhalation" and "Exhalation" cycles or after both, as explained above under c) and d). It allows the replacement of new gas or oxidant compound.

It is advisable to control the temperature of the process of " Controlled Breathing ". Temperature variations while the container pressure is monitored very sensitively can improve treatment outcomes. It is also possible to control and vary the temperature of the incoming gas regarding the product so that there is a differential between the two.

A forced circulation of the atmosphere inside the container, with the application of pressure oscillations will improve pressure distribution. At the same time, you can leverage to increase production of ozone from oxygen in the inner atmosphere, if we force it to recirculate in the area of the circuit where ozone-generating devices are located.

In this specification, when the term "filter" is used, we mean any porous element made of any material, whether sieves, sleeves, screens, meshes, etc. or a combination of them; the important thing is that their porosity is inferior to the granulometry of the product being processed.

It is advisable to locate these filters adjacent to the cork to be treated, both in the area of the intake and gas extraction, because it will facilitate greater contact and uniform pressure distribution. It is also advisable to strengthen and support the filters for at least one of its sides, with a piece of metal with higher porosity to frame them.

The filters in the gas extraction area may be partially clogged due to the small particle size of some derivatives cork. To avoid this problem, the pressures and/or sweeps between zones intake / extraction will be regularly and by default inverted, alternating their functionality to reverse the direction of gas flow.

It should be noted that another function of the filter is to retain in the treatment chamber small particles, thereby making it impossible for product to be lost.

It is also recommended to control -through adding or extraction- humidity within the container to prevent excessive drying or wetting.

It is also feasible to provide steam (of any kind, wet, dry, etc.) to the treatment. It helps control temperature and humidity more easily and it's beneficial to improve the reaction of oxidizing agent introduced. Its application can be included at any step in the process of "Controlled Breathing" and/or sweep.

It is also possible to controllably dry the product while the method "Controlled Breathing" applies. This will need a cold machine (refrigerator) sealed to communicate with the tank and control the temperature and humidity inside. An induced air recirculation allows the moisture to condense on their interaction with cold batteries, controllably removing the condensation obtained in the drying treatment. We achieve controlled drying and improvement in the cork's moisture.

### Drawings description

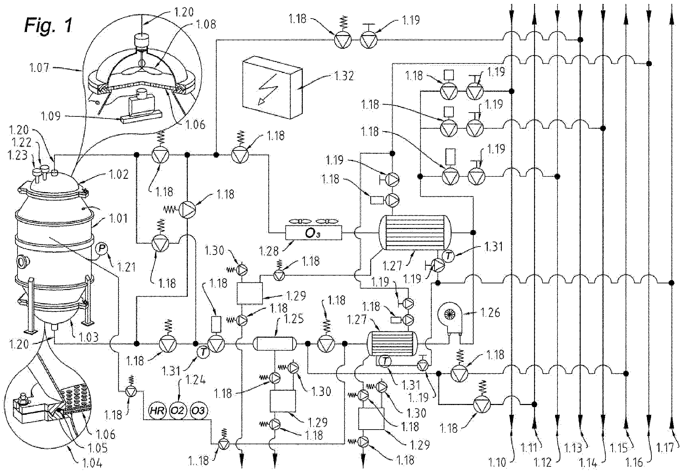

Figure 1, perspective view of the airtight container and the additional equipment required to perform the treatment.
- Nº1.01: Gastight container, (with thermal insulation surrounding its structure, not shown in the drawing)
- Nº1.02: Airtight and hinged flap for filling the product.
- Nº1.03: Airtight and hinged flap for emptying the product.
- Nº1.04: Inside detail of the emptying, with the same mechanism of filtering and support as the higher gate, but the latter, in the reverse position.
- Nº1.05: Sealing gasket.
- Nº1.06: Sandwich structure formed by a perforated plate at the top, a sieve filter in the intermediate zone and a lower perforated plate with mechanical capacity to withstand and product assembly. The same format is valid for mounting the upper loading gate.
- Nº1.07: Inside detail of the filling gate.
- Nº1.08: Rotary type diffuser for homogeneous distribution in the upper air chamber, which is also installed in the lower air chamber.
- Nº1.09: Vibrations generator of mechanical waves.
- Nº1.10: General oxygen line.
- Nº1.11: Gases exit line to the outside.
- Nº1.12: General air admission duct at atmospheric pressure.
- Nº1.13: General vapour duct.
- Nº1.14: General compressed air duct.
- Nº1.15: General vacuum duct.
- Nº1.16: General duct for cold-water drive.
- Nº1.17: General cold-water return duct.
- Nº1.18: Pilot valves that control fluid throughout the process.
- Nº1.19: Manually operated switching valves.
- Nº1.20: Flexible pipe for connection to the gates.
- Nº1.21: Pressure sensor.
- Nº1.22: Overpressure safety valve.
- Nº1.23: Depressions safety valve.
- Nº1.24: Set of chemical sensors.
- Nº1.25: Airtight container for lung effect.
- Nº1.26: Turbine for gas recirculation.
- Nº1.27: Heat exchanger.
- Nº1.28: Ozone Generator.
- Nº1.29: Set of elements for condensate discharge.
- Nº1.30: Pilot operated valve connected to atmosphere and the water trap, used to facilitate evacuation.
- Nº1.31: Temperature sensor.
- Nº1.32: Switchboard where the process is monitored and controlled.

### Detailed description of production examples

Although the drawing shows a watertight and resistant to pressures vessel, insulated to heat, handy and mobile (1.01), the application can have multiple of them, arranged in an installation that allows them to work in series or individually in the treatment of "Controlled Breathing". The advantage of this system lies in its simplicity because it ignores important container volumes, which require significant specific areas, with the consequent economic savings. Also, the containers are easy to handle and have great versatility that can treat various cork derivatives in each container. With the ability to add or remove elements to the process we also gain flexibility and dynamism.

These containers shall be, preferably, circular (1.01) to withstand better the pressures, and they can carry at least one metal reinforcing ring welded on its outer perimeter. Also they will have a sealed door access for maintenance, which can be the filler and/or emptying. At least one of its parts, the top or bottom, shall be conical, in this example they both are.

The loading and unloading operations are performed through folding or hinged lids that seal the container at the top (1.02) and bottom (1.03). Each cover has at least one filter, sieve or mesh (1.06) of lower porosity than the product to be treated, which are secured by at least one perforated plate, which in turn serves as support for the product. These filters / sieves / meshes have a plurality of holes or porosities for homogenizing pressures and to prevent the outflow of particles; its porosity is changed to suit the product.

The valves that process the method are of two types;
a) (1.18) may be for closing and opening (all or nothing) and/or type "servo piloted" of proportional opening and closing with drive and capacity regulated by computer control monitoring. Both can be combined in this process.
b) The valves (1.19) are closed manually.

In this example, the process begins at a controlled temperature with values ranging between 1 °C and 20 °C.

The container also has at least one pressure sensor (1.21) and further items for monitoring and controlling the oxidizing agent added and/or consumed (1.24) may be included; as well as for generated chemicals control. Its function is to control the "Inhalation", "Exhalation" and/or sweeping, ultimately, the process of "Controlled Breathing", but also the combination of the valve opening and closing (all or nothing) and/or proportional servo piloted with controlled opening and closure (1.18) that connect the container with the pressurization's general circuits, as illustrated in the drawing. This control can be managed individually for each container by monitoring the computer system (1.31). Thus we have the advantage of treating different format and volume corks in each container. Intercalating its function provides significant energy savings.

Unlike with pressure sensors, it is possible to control the oxidizing agent added and/or consumed, or for chemicals which being generated to be installed independently (1.24), this way, various containers at a time could be controlled. It would only be necessary to establish a communication system between each sensor and the containers it controls. This collection of individualized data is commuted alternatively sequentially under the rule of the computer system, which would open and shut the valves interposed between sensor and containers, while it would monitor the collected data. The advantage of this option is the savings in cost achieved by installing fewer sensors.

The containers described above are installed and connected in the infrastructure that serves to manage the treatment. This includes at least the following circuits:
a) Administration of pressurized gas (atmospheric gas with 02, with the option of adding 02, 03 and/or fluid humidity control).
b) Administration of gas at atmospheric pressure with controlled humidity, with or without ozone and with or without oxygen enrichment.
c) Vacuum.
d) Depressurization.
e) Recirculation of fluid via a tight gas pump, with or without generating ozone with temperature and humidity control which links with another loop circuit.
f) Cooling circuit recirculation (cold / hot), which links to the other loop circuit to control the product's temperature.
g) Administration of heat fluid by through heat and/or steam generators.

Each of the circuits (1.10-1.17), for example, is formed by a metal pipe from which an extension of circuits bifurcate to finally link to the top of the container and another to the bottom of it, each one with its respective valve. These bifurcations are connected to the upper and lower distributors /collectors (1.19). From here, the link with the two ends of the container is made by means of two flexible tubes (1.20). The direct connection with the loading and unloading articulated or hinged covers is done through servo piloted operated valves (1.18) to control the pressure and volume of the container and pressure oscillations.

As described above, thanks to the controlled pressure distribution, the system allows a sweep in the selected containers and to control the flow and sequentially reverse fluid. We can do this by combining some of the circuits 1.10 - 1.17. The aim is to deobturate membranes or filters of micro particles that could have possibly adhered and to homogenize the treatment.

After that, corks are added and the temperature is controlled at about 12 °C. Then, reversing the flow direction after a predetermined time performs a sweep up and down. Subsequently a vacuum of 10-mbar is performed. Atmospheric gas is added passing through the ozone generator (1.28) until reaching predetermined concentration and pressure values. Now a recirculation of the added gas is performed, forcing it to pass through the ozone generator while absolute pressure of 1,275 to 1,300 mbar oscillations are performed during predetermined times. It is significant to note that the cork has large porosities that require an exposure time to pressure to absorb it. This absorption causes a drop in pressure in the container that must be compensated by adding more fluid at a controlled pressure.

These pressure levels are maintained and consumptions of 03 and 02 are monitored until they reach predetermined levels. This cycle is repeated until pre-programmed solutions are reached. Once this point is reached, we proceed to vacuum at 5 mbar, to then introduce steam at a temperature of 100 °C, leaving the valve of the opposite gate open to extract steam and avoid over-pressure to the product. Alternatively the steam flow will be reversed to homogenize the process. The treatment consists on repeating the cycle phases of "Controlled Breathing" controlled by the relative consumption of oxidizing agent or by increase and/or decrease in chemicals.

## Claims

1. Method of treatment comprising its application to all kinds of compounds, substances and cork products, in their natural state or in all and each of its stages of development or manufacture. This method is **characteristic** because elements to treat are arranged inside a container or sealed circuit, they are subjected to a temperature ranging between 1 and 20, and are induced pressure variations by providing a gas or gas mixture, which have oxidizing properties to be introduced or dissolved in Cork. Subsequently the gases are extracted so that at least one "Controlled Breathing" cycle is completed with the following steps:
a). A gas, with an oxidant part, pressure differential is introduced.
b). Increased pressure is applied, by providing a gas with predetermined composition.
c). Gas recirculation is applied within the container forcing it to go through an ozone generator and / or a gas with an oxidant part is administered periodically.
d). In parallel to step a) and / or b) and / or c), the amount present in the medium of at least one of the chemicals supplied and / or generated in the reaction is monitored and / or controlled, so that, once an increase or decrease of the default value is detected, the next step starts.

2. Method according to claim 1, **characteristic** because a vacuum and / or controlled sweep in the atmospheric gas inside the container is applied, allowing removal and / or renewal, simultaneously providing an oxidizing agent.

3. Method, according to claim 1, **characterized by** the induction of pressure oscillations that span the range of atmospheric values and higher and lower.

4. Method, according to claim 1, **characterized by** the control of the length of steps a), b) and c) via a timing system.

5. Method, according to claim 1, **characterized by** the inclusion of multiple repetitions of the cycles during all the process, until reaching predetermined final chemical parameters.

6. Method, according to claim 1, **characterized by** the monitoring the reduction and / or increase of the initial provided oxidizing agent.

7. Method, according to claim 1, **characterized by** its communication with the interior volume of the container and the surrounding outside atmosphere by opening at least one gate to cause the container to recover the interior atmospheric pressure's values to default ones.

8. Method, according to claim 1, **characterized by** a controlled force to renew the composition of the internal atmosphere, by vacuum suction system with controlled opening of at least one gate positioned substantially at the opposite end to the aspiration place.

9. Method, according to claim 1 **characterized by** a controlled sweep to renew the composition of the internal atmosphere that is forced by administering a combined overpressure with controlled opening of at least one gate located on the substantially opposite end of the administration place.

10. Method, according to claim 1, **characterized by** the selection of the oxidizing gas applied between ozone and oxygen is selected.

11. Method, according to claims 1 and 10, **characterized by** the use of atmospheric oxygen gas.

12. Method, according to claims 1 and 10, **characterized by** the generation of used ozone in the environment and / or site.

13. Method, according to claim 1, **characterized by** the control within the container of the direction of the gas flow.

14. Method, according to claims 1 and 13, **characterized by** that the alternation of function between the places of introduction and extraction of gas to reverse the direction of gas flow.

15. Method, according to claim 1, **characterized by** the application of vibrations to cause pressure waves.

16. Method, according to claim 1, **characterized by** the control of gas humidity when introduced into the container.

17. Method, according to claim 1, **characterized by** the control of temperature and / or humidity and / or recirculation of the atmosphere inside the container.

18. Method, according to claim 1, **characterized by** the application of at least one temperature change to rise the temperature to more than 20 ° C

19. Method, according to claims 1 and 18, **characterized by** the application of temperature change in the process by generating heat and / or steam.

20. Method, according to claim 1, **characterized by** the passing of the re-circulated gas through at least one heat/cold exchanger.

21. Installation for the treatment of cork products through a "Controlled Breathing" process, comprising a container sealing, with at least a loading and unloading door- built to contain the material to be treated, **characterized** because it has at least one chemicals sensor, and, located at substantially opposite ends of the installation, it includes at least two mechanical assemblies, each of which contains:
a) Pressure and vacuum controlled systems.
b) At least one controlled opening connected to the outside, used for purging pressure and / or the generation of a sweep.
c) At least one set of filters or sieves, adjacent to the material to be treated.
The mechanical location of each set forces an administered fluid path, which crosses in this order:
d) The filter / sieve adjacent to the flow inlet duct.
e) The material to be treated.
f) Outlet ducts substantially opposite to the inlet arrangement.
The installation has means for controllably reversing the direction of the flow path of the gases inside the container.

22. Installation, according to claim 21, **characterized by** the incorporation of at least one connection with at least one ozone and / or oxygen generator.

23. Installation, according to claim 21 and 22, **characterized by** that it comprises an ozone generator which is associated with at least one of the inputs of the fluid, where a pressure differential which causes the sweep is generated.

24. Installation, according to claim 21 and 22, **characterized by** the incorporation of an ozone generator that is associated with the recirculation circuit.

25. Installation, according to claim 21, **characterized by** having at least one chemical analyser which captures data decreases and / or increases in one of the oxidising agents administered; as well as those generated in reaction.

26. Installation, according to claim 21, **characterized by** counting with at least one heat/cold exchanger where gases circulate.

27. Installation, according to claim 21, **characterized by** the inclusion of a static container.

28. Installation, according to claim 21, **characterized by** having means to control the temperature, humidity and the inner atmosphere recirculating installation.

29. Installation, according to claim 21, **characterized by** having ducts for recirculating has the inner atmosphere of the installation.

30. Installation, according to claim 21, **characterized by** the incorporation of a vibrator.

31. Use of the method **characterized by** the use for removal of 2-4-6 trichloroanisole (TCA) from cork.
